Europäisches Patentamt

European Patent Office

Office européen des brevets

(11) Veröffentlichungsnummer: **0 075 886**
**A2**

# (12) EUROPÄISCHE PATENTANMELDUNG

(21) Anmeldenummer: 82108847.3

(22) Anmeldetag: 24.09.82

(51) Int. Cl.³: **C 12 P 1/00**
C 12 N 5/00, C 12 N 15/00
//C12R1/91

(30) Priorität: 25.09.81 DE 3138230

(43) Veröffentlichungstag der Anmeldung:
06.04.83 Patentblatt 83/14

(84) Benannte Vertragsstaaten:
AT BE CH DE FR GB IT LI LU NL SE

(71) Anmelder: Max-Planck-Gesellschaft zur Förderung der Wissenschaften e.V.
Bunsenstrasse 10
D-3400 Göttingen(DE)

(72) Erfinder: Lohmann-Matthes, Marie-Luise, Prof. Dr.
Laengenhardtstrasse 16
D-7800 Freiburg(DE)

(72) Erfinder: Sun, Deming, Dr.
Ettenheimerstrasse 22
D-7800 Freiburg(DE)

(74) Vertreter: Weickmann, Heinrich, Dipl.-Ing. et al,
Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22
D-8000 München 86(DE)

(54) Verfahren zur Gewinnung von Makrophagen-Aktivierungsfaktor (MAF).

(57) Ein Verfahren zur Gewinnung von Makrophagen-Aktivierungsfaktor ist dadurch gekennzeichnet, daß man mit Concanavalin A oder Phytohämaglutinin stimulierte T-Zellen aus Milz oder peripherem Blut von Maus und Mensch züchtet und den Makrophagen-Aktivierungsfaktor aus den Kulturüberständen gewinnt.

EP 0 075 886 A2

Croydon Printing Company Ltd.

- 1 -

Verfahren zur Gewinnung von Makrophagen- Aktivierungsfaktor (MAF)

Die Erfindung betrifft ein Verfahren zur Gewinnung
von Makrophagen-Aktivierungsfaktor.

Es ist bekannt, aus Lymphzellen einen die Makrophagen-
Zytotoxizität aktivierenden Faktor zu gewinnen (MAF).
Es handelt sich hierbei um ein hochaktives Lymphokinmaterial, welches auch als MCF bezeichnet wurde. MAF aktivierte Makrophagen haben die Fähigkeit, Tumorzellen
zu zerstören. MAF hat daher sowohl wissenschaftliches
als auch therapeutisches Interesse.

Einer gewerblichen Verwertung dieses Materials steht
entgegen, daß bisher kein Verfahren existiert, welches
die Herstellung der benötigten Mengen gestatten würde.

Aufgabe der Erfindung ist es daher, ein neues Verfahren
zur Gewinnung von MAF zu schaffen, welches wesentlich
größere Mengen an MAF zu gewinnen gestattet und damit
diese Substanz der gewerblichen Verwendung zugänglich
macht.

Gelöst wird diese Aufgabe erfindungsgemäß durch ein Verfahren zur Gewinnung von Makrophagen-Aktivierungsfaktor, welches dadurch gekennzeichnet ist, daß man mit Concanavalin A oder Phytohämaglutinin stimulierte T-Zellen aus Milz oder peripherem Blut von Maus oder Mensch züchtet und aus den Kulturüberständen Makrophagen-Aktivierungsfaktor gewinnt.

Dabei ist eine bevorzugte Ausführungsform ein Verfahren, das dadurch gekennzeichnet ist, daß man mit Concanavalin A oder Phytohämaglutinin stimulierte T-Zellen aus Milz oder peripherem Blut von Maus und Mensch mit einer Maus-Lymphomzellenkultur fusioniert und aus den erhaltenen Klonen diejenigen auswählt und weiterzüchtet, welche bei Stimulierung mit Concanavalin A oder Phytohämaglutinin Makrophagen-Aktivierungsfaktor produzieren und letzteren aus den Kulturüberständen dieser Klone gewinnt.

Dieses bevorzugte Verfahren der Erfindung beruht auf der überraschenden Feststellung, daß Hybridom-Kulturen aus T-Blasten, die aus der Milz oder dem peripheren Blut stammen und Lymphomzellen bei Stimulierung mit Concanavalin A oder Phytohämaglutinin eine mehr als hundertfach stärkere Bildung von MAF, bezogen auf Zellzahlbasis, ergeben, als die bisher verwendeten Zellen.

Vorzugsweise führt man das erfindungsgemäße Verfahren so durch, daß man die T-Zellen mit 0,5 bis 50 µg/ml Concanavalin A oder Phytohämaglutinin in einem geeigneten Zellwachsmedium 30 bis 60 Stunden stimuliert, dann entweder sofort oder nach 4 bis 10 Tagen Kultur in einem eine organische SH-Verbindung und Interleukin II enthaltenden Medium kultiviert, die so erhaltene T- Blasten-Kultur mit einer Lymphomzellen-Kultur mischt und in an sich bekannter Weise
fusioniert, die aus den fusionierten Zellen erhaltenen
Klone 15 bis 48 Stunden mit 0,5 bis 5 µg Concanavalin
A oder Phytohämaglutinin pro ml Kulturlösung stimuliert und danach aus dem Kulturüberstand das Produkt
gewinnt. Concanavalin A oder Phytohämaglutinin können
entweder während der gesamten Produktionsphase von
MAF im Kulturmedium sein, oder sie können nach
mindestens 30 Minuten Kontakt aus der Kultur durch
Waschen entfernt werden.

Als Medium für die Stimulierung der T-Blasten wird
vorzugsweise Eagle's Medium (Virology 8, 396 (1959))
oder RPMI 1640 (J.N.C.I. 22, 1003, 1959) verwendet.
Vorzugsweise wird diesem Medium noch Serum zugesetzt.
Besonders gute Ergebnisse erhält man bei Verwendung
von foetalem Kälberserum. Die T-Blasten-Konzentration
liegt zweckmäßig bei etwa 1 bis 20 x $10^6$ Zellen pro
ml.

Nach der Stimulierungsphase mit Concanavalin A erwies
es sich als zweckmäßig, eine Konzentration der
T-Blasten durch Dichtegradienten durchzuführen, ehe
entweder die Fusion sofort stattfindet oder die Kultivierung weitere 4 bis 10 Tage fortgesetzt wird unter
Verwendung von zweckmäßig dem gleichen Medium wie in
der Stimulierungsphase.

Als SH-Verbindung wird Merkaptoethanol (5 x $10^{-5}$m)
bevorzugt. Der Interleukin II Zusatz (J. of Immunology
120, 2027 (1978)) liegt zweckmäßig in der Größenordnung
von 10 bis 40 %.

Die Fusion der Zellen erfolgt im Prinzip nach bekannten Methoden, wie sie von Köhler und Milstein (Eur. J. Immunol. 1976, 6, 511) beschrieben wurden. Hierzu werden die zu fusionierenden Kulturen nach Waschen in serumfreiem Medium vermischt und zusammen abzentrifugiert. Das erhaltene Sediment wird wieder in serumfreiem Medium suspendiert und vorsichtig mit einer Mischung von Polyäthylenglycol und Dimethylsulfoxid versetzt. Nach kurzer Einwirkungsdauer, in der Regel etwa 5 Minuten, wird das Gemisch wieder in serumfreiem Medium gewaschen. Es enthält nunmehr die fusionierten Zellen und wird auf Gewebekulturlochplatten aufgeteilt und in einem geeigneten Medium wie Eagle's Medium oder RPMI 1640 kultiviert, bis sichtbare Klone auftreten.

Die erhaltenen Klone werden wiederum serumfrei mit Concanavalin A oder Phytohämaglutinin stimuliert. Geeignete Konzentrationen sind 0,5 bis 10 µg/ml. Unter diesen Bedingungen ergeben die Zellen der Klone den gesuchten MAF an das Medium ab. Das Medium kann als solches nach Abtrennung der Zellen verwendet werden. Normalerweise ist es auch möglich, MAF daraus abzutrennen, beispielsweise durch hydrophobe Chromatographie, Gel-Filtration und dgl., Fällung mit organischen Lösungsmitteln und nach den üblichen biochemischen Reinigungsmethoden für Proteingemische aufzureinigen. Bei der Reinigung wendet man zur Feststellung der MAF-haltigen Fraktionen einen Test an, wie er im Beispiel beschrieben ist. Er beruht auf der Zerstörung von Tumorzellen, die $^{51}$Chrom enthalten und dieses bei der Zerstörung in die Lösung abgeben, wo es bestimmt werden kann.

Eine weitere bevorzugte Ausführungsform des erfindungsgemäßen Verfahrens ist dadurch gekennzeichnet, daß man
T-Zellen mit 0,5 bis 50 µg/ml Concanavalin A oder
Phytohämaglutinin in einem geeigneten Zellwachsmedium
30 bis 60 Stunden stimuliert und dann die erhaltenen
T-Blasten sofort oder nach 4 bis 10 Tagen weiterer
Kultur in einem Kulturmedium, das 10 bis 30 % Interleukin II enthält, weiterkultiviert. Am 3. bis 8. Tag
nach der Weiterkultivierung in interleukinhaltigem
Medium werden 55 bis 85 % der erhaltenen T-Blasten
der Kultur entnommen und mit Concanavalin A oder
Phytohämaglutinin stimuliert, die verbliebenen 15 bis
45 % der T-Blasten weiterkultiviert und nach jeweils
3 bis 8 Tagen diesen Vorgang bis zu 10 Wochen lang
wiederholt.

Bei dieser bevorzugten Ausführungsform, bei der die
nach Stimulierung erhaltenen T-Blasten in einem Kulturmedium, das 10 bis 30 % Interleukin II enthält, weiterkultiviert werden, vermehren sich die T-Blasten während
der Kultur in diesem Medium ganz außerordentlich stark.
Daher werden ab dem 3. bis 8. Tag, vorzugsweise am 5.
oder 6. Tag, dieser Kultur 55 bis 85 %, vorzugsweise 70
bis 80 % der erhaltenen T-Blasten entnommen und mit
Concanavalin A oder Phytohämaglutinin stimuliert. In
serumhaltigem und auch in serumfreiem Medium produzieren diese T-Blasten dann große Mengen von Makrophagen-
Aktivierungsfaktor und auch von Interleukin II und
anderen Lymphokinen. Das hierbei gewonnene Interleukin
II wird verwendet zur weiteren Kultivierung der
verbliebenen 15 bis 45 % der T-Blasten. Dieser verbliebene Teil der T-Blasten wiederum expandiert nach 3 bis
8 Tagen. Dann wird derselbe Vorgang wiederholt. Dieses
Verfahren läßt sich bis zu 10 Wochen lang weiterführen,
vorzugsweise wird es 4 bis 8 Wochen lang durchgeführt.

Auf diese Weise ist es möglich, z. B. von 500 ml Blut statt, nach herkömmlicher Methode 50 ml Makrophagen-Aktivierungsfaktor-haltigen Überstand, mindestens 1000 ml zu produzieren.

Wird als Ausgangsmaterial Zellmaterial aus der Milz verwendet, so können statt 6 1 Überstand 60 bis 100 1 Überstand produziert werden. Dieses Verfahren eignet sich also zur Gewinnung großer Mengen von Makrophagen-Aktivierungsfaktor aus einer kleinen Menge an Zellausgangsmaterial.

Weiterhin hat es den Vorteil, daß unabhängig von der Hybridisierungstechnik und den permanenten Hybridom-Kulturen ständig voneinander unabhängig neue Produktionsansätze gemacht werden können. Die Effizienz dieses Verfahrens beruht auf der in-vitro-Anreicherung und Expansion Makrophagen-Aktivierungsfaktor produzierender T-Blasten.

Das erfindungsgemäße Verfahren ermöglicht es, MAF in großer Menge zu bilden und damit der gewerblichen Verwertung zugänglich zu machen. Dabei kann entweder die Produktion an MAF auf die mehrhundertfache Menge gesteigert werden oder aber, bei einer Steigerung um das zehn- bis zwanzigfache, MAF unabhängig von der Hybridisierungstechnik gebildet werden.

Die folgenden Beispiele erläutern die Erfindung.

Beispiel 1

C57BL/10 oder AKR Milzzellen werden mit 5 μg Concanavalin A für 48 Stunden stimuliert. Dabei lagen $5 \times 10^6$ Milzzellen pro ml in Eagle's Medium mit 10 % feotalem Kälberserum vor. Nach 48 Stunden wurden die Milzzellen über einem Dichtegradienten (Ficoll/Hypaque) gegeben, wobei sich die Blasten der Kultur in der Interphase stark anreichern. Diese Blasten wurden in einer Konzentration von $1 \times 10^6$/ml weitere 8 Tage in Eagle's Medium mit 10 % feotalem Kälberserum, das mit $5 \times 10^{-5}$ Merkaptoethanol und 30 % Interleukin II (J. of Immunology 120, 20-27 (1978)) supplementiert war, kultiviert. In diesem Zeitraum erfolgte eine Stabilisierung der T-Blasten-Kultur. Die Klonierungs-Effizienz dieser in interleukinhaltigen Medien 8 Tage weitergezüchteten T-Blasten ist wesentlich höher als wenn die Fusion direkt nach der anfänglichen 48-stündigen Stimulierung mit Concanavalin A oder Phytohämaglutinin stattfindet.

$100 \times 10^6$ T-Zell-Blasten aus den obigen Kulturen wurden dreimal mit serumfreiem Medium gewaschen. Ebenso wurden $10 \times 10^6$ Zellen des HAT-sensitiven AKR-T-Lymphoms BW 5147 (J. of Imm. 29, 353 (1979)) dreimal gewaschen. Die beiden Zellpopulationen wurden dann in 50 ml Kunststoffröhrchen vermischt und 3 Minuten zentrifugiert. Das Sediment wurde in 0,2 ml serumfreiem Medium resuspendiert und tropfenweise mit 1 ml 30 %igem Polyäthylenglycol MG etwa 4000 und 10 % Dimethylsulfoxid versetzt. Nach 5 Minuten wurde das Reaktionsgemisch dreimal in serumfreiem Medium gewaschen. Das Sediment, welches nun fusionierte Zellen enthält, wurde in 300 ml Eagle's Medium (Dulbeccos modification) + 15 % feotales Kälberserum (Virology 8, 396 (1959)) aufgenommen und in 2 ml Portionen auf Gewebskulturlochplatten aufgeteilt. Nach 24 Stunden wurde zu jeder Probe 1 ml HAT-Medium (Eur. J. of Immunology 6, 511 (1967)) gegeben. Jeden 2. Tag wurde 1 ml Medium durch 1 ml HAT-Medium ersetzt. Etwa 5 Tage nach der Fusion wurden die ersten Klone sichtbar.

Zwischen dem 10. und 20. Tag wurde von jedem Klon ein Duplikat durch Teilung und Überimpfung hergestellt und die Klone wurden mit 2 µg Concanavalin A pro ml in serumfreiem Medium 24 Stunden stimuliert. Die nicht stimulierten Duplikat-Kulturen wurden bis zur Beendigung des nachstehend beschriebenen funktionellen Tests gefüttert.

Funktioneller Test:

$2 \times 10^5$ Maus-Makrophagen wurden in 96 Vertiefungen einer Falkon-Mikrotiter-Platte eingefügt. Jeder Vertiefung wurden 0,1 ml Überstand der stimulierten Klon-Kulturen zupipettiert. Nach 24 Stunden wurden die Kulturen zweimal gewaschen und jedem Loch $1 \times 10^4$ $^{51}$Chrom Test-Tumorzellen gegeben. Makrophagen, die mit aktiven Hybridom-Überständen aus den stimulierten Klonkulturen inkubiert waren, waren nach 24 Stunden aktiviert und nun imstande, die Tumorzellen zu zerstören.

Die Zerstörung wird gemessen als Freisetzung des $^{51}$Chrom aus den zerstörten Tumorzellen in den Überstand. Nur diejenigen Hybridom-Kulturen, die auf die Concanavalin-A-Stimulation hin MAF produzieren, wurden weiter kultiviert. Diese Kulturen erwiesen sich seit 6 Wochen als stabil und produzieren große Mengen MAF (als hochaktives Lymphokin-Material). $5 \times 10^5$ Zellen einer derartigen Linie produzieren zwanzigmal mehr MAF wie $10 \times 10^6$ unfusionierte Milzzellen. Die MAF-Produktion beträgt daher etwa das 400-fache bezogen auf Zellzahl als Basis.

Beispiel 2

Lymphocyten aus menschlicher Milz wurden 48 Stunden mit 5 µg Concanavalin A stimuliert. Dann wurden die erhaltenen T-Blasten in einem Medium, das 10 % Interleukin II enthielt, weiterkultiviert. Dabei vermehrten sich die T-Blasten sehr stark. Am 6. Tag dieser Kultur wurden 75 % der erhaltenen T-Blasten der Kultur entnommen und mit Concanavalin A stimuliert. Diese restimulierten

Blasten wurden dann in serumhaltigem Medium weiterkultiviert. Dabei produzieren diese T-Blasten große Mengen an Makrophagen-Aktivierungsfaktor, an Interleukin II und an anderen Lymphokinen. Das dabei gewonnene Interleukin II kann wieder verwendet werden zur weiteren Kultivierung. Die in dem Kulturmedium, das 10 % Interleukin II enthielt, verbliebenen 25 % T-Blasten wurden nun 3 Tage lang weiter expandiert.

Derselbe Vorgang wurde wiederholt. Dieses Verfahren wurde 8 Wochen lang weitergeführt. Dabei wurden 80 l lymphokinhaltige Überstände erhalten.

P a t e n t a n s p r ü c h e

1.  Verfahren zur Gewinnung von Makrophagen-
    Aktivierungsfaktor,     d a d u r c h
    g e k e n n z e i c h n e t ,     daß man mit Con-
    canavalin A oder Phytohämaglutinin stimulierte
    T-Zellen aus Milz oder peripherem Blut von Maus
    und Mensch züchtet und den Makrophagen-Aktivierungsfaktor aus den Kulturüberständen gewinnt.

2.  Verfahren nach Anspruch 1,     d a d u r c h
    g e k e n n z e i c h n e t ,     daß man die mit
    Concanavalin A oder Phytohämaglutinin stimulierten
    T-Zellen aus Milz oder peripherem Blut von Maus
    und Mensch mit einer Maus-Lymphomzellenkultur fusioniert und aus den erhaltenen Klonen diejenigen
    auswählt und weiterzüchtet, welche bei Stimulierung mit Concanavalin A oder Phytohämaglutinin
    Makrophagen-Aktivierungsfaktor produzieren, und
    letzteren aus den Kulturüberständen dieser Klone
    gewinnt.

3.  Verfahren nach Anspruch 2,     d a d u r c h
    g e k e n n z e i c h n e t ,     daß man die T-
    Zellen mit 0,5 bis 50 µg/ml Concanavalin A oder
    Phytohämaglutinin in einem geeigneten Zellwachsmedium 30 bis 60 Stunden stimuliert, dann entweder
    sofort oder nach 4 bis 10 Tagen weiterer Kultur in
    einem, eine organische SH-Verbindung und Interleukin II enthaltendem Medium die so erhaltene T-
    Blasten-Kultur mit einer Lymphomzellen-Kultur
    mischt und in an sich bekannter Weise fusioniert,
    die aus den fusionierten Zellen erhaltenen Klone 15 bis 48 Stunden mit 0,5 bis 5 µg Concanavalin A oder Phytohämaglutinin pro ml Kulturlösung stimuliert und danach aus dem Kulturüberstand das Produkt gewinnt.

4. Verfahren nach einem der vorhergehenden Ansprüche, d a d u r c h   g e k e n n z e i c h n e t , daß man die T-Blasten in einem serumhaltigen Medium kultiviert.

5. Verfahren nach Anspruch 4,   d a d u r c h   g e k e n n z e i c h n e t ,   daß man feotales Kälberserum verwendet.

6. Verfahren nach einem der vorhergehenden Ansprüche, d a d u r c h   g e k e n n z e i c h n e t , daß man einer Mischung von T-Blasten und Lymphomzellen in serumfreiem Medium Polyäthylenglycol und Dimethylsulfoxid zusetzt und 2 bis 10 Minuten einwirken läßt.

7. Verfahren nach einem der vorhergehenden Ansprüche, d a d u r c h   g e k e n n z e i c h n e t , daß man einen Tag nach der Fusion HAT-Medium zusetzt.

8. Verfahren nach Anspruch 1,   d a d u r c h   g e k e n n z e i c h n e t ,   daß man T-Zellen mit 0,5 bis 50 µg/ml Concanavalin A oder Phytohämaglutinin in einem geeigneten Zellwachsmedium 30 bis 60 Stunden stimuliert, dann die erhaltenen T-Blasten sofort oder nach 4 bis 10 Tagen weiterer Kultur in einem Kulturmedium, das 10 bis 30 % Interleukin II enthält, weiterkultiviert, nach dem 3. bis 8. Tag 55 bis 85 % der erhaltenen T-Blasten der Kultur entnimmt und mit Concanavalin A oder Phytohämaglutinin stimuliert, die verbliebenen 15 bis 45 % der T-Blasten weiterkultiviert in einem Medium, das 10 bis 30 % Interleukin II enthält, und nach jeweils 3 bis 8 Tagen den Vorgang bis zu 10 Wochen lang wiederholt.